# EUROPEAN PATENT SPECIFICATION

(11) **EP 2 635 208 B1**
(45) Date of publication and mention of the grant of the patent: **08.10.2014**
(21) Application number: 11794234.2
(22) Date of filing: 02.11.2011
(51) Int. Cl.: A61B 17/32

(54) **BLUNT DISSECTOR**
STUMPFER SEZIERER
DISSECTEUR ÉMOUSSÉ

(30) Priority: 03.11.2010 WO PCT/IB2010/002805
(43) Date of publication of application: 11.09.2013
(73) Proprietor: Barokas, Albert, 1206 Geneva (CH); Damascelli, Bruno, 20133 Milano (IT)
(72) Inventor: Barokas, Albert, 1206 Geneva (CH); Damascelli, Bruno, 20133 Milano (IT)
(74) Representative: Cronin, Brian Harold John
(86) International application number: PCT/IB2011/054867
(87) International publication number: WO 2012/059870

(56) References cited:
- BE-A6- 1 006 811
- US-A- 6 036 713
- US-A1- 2009 234 379
- US-B1- 6 264 670

## Description

### BACKGROUND OF THE INVENTION

### 1. Field of the Invention

The present invention pertains to the field of surgery and surgical instruments. More specifically, the present invention discloses a novel blunt dissector for creating cavities within tissues of the human or animal body, or between tissue plans of the same. Such newly created cavities may notably be used for placing medical devices or any other foreign bodies.

### 2. Description of the Prior Art

During surgical operations, physicians have to open the skin and then penetrate the tissues or dissect tissue plans in order to reach the anatomical area where a specific surgical procedure has to take place. In order to reach this anatomical area, the physician may use blade scalpels, electric scalpels, his own fingers or any instrument able to penetrate tissues or dissect tissue plans. Whenever the specific surgical procedure is to create a cavity within tissues or between tissue plans, the physician has to cut or dissect an area whose size is appropriate for the purpose of the cavity.

Whenever the cavity is created in view of inserting a foreign body such as a medical device, the appropriate size of the cavity is determined by the size of the foreign body. The physician uses the instruments and techniques described above to create the cavity, enlarging the pathway initially created within the tissues or between the tissue plans.

Creating the cavity with surgical instruments or techniques (own finger) available in the prior art is time consuming, traumatic, inaccurate as to the exact location and size of the cavity, and sometimes difficult. The resulting cavity may be too large or poorly located for the intended purpose, excessive bleeding requiring hemostasis may occur due to unwanted trauma or preexisting coagulation problems, and the whole procedure is time-consuming, which increases stress for the patient. In addition, depending on the location and anatomical depth of the cavity, the cavity may also be quite difficult to create in the first place.

Therefore, there is a need for a novel surgical instrument allowing the operator to penetrate tissues, dissecting tissue plans and create cavities within the human or animal body in an easy, accurate, fast and non-traumatic manner.

Document US 6036713 A, which forms the basis of the preamble of claim 1, discloses a surgical instrument for creating a cavity in the human or animal body.

### SUMMARY OF THE INVENTION

The present invention provides a hand-held surgical instrument which allows to overcome the above-mentioned drawbacks of surgical instruments and techniques of the prior art. The surgical instrument according to the invention is a blunt dissector for penetrating tissues, dissecting tissue plans and creating cavities within the human or animal body accurately, in an expedited way and with minimum trauma and risk. Such cavities may be used for placing a medical device or for any other purposes. The surgical instrument according to the invention may be executed in different sizes in order to accommodate various surgical procedures and/or different medical devices or other foreign bodies. After an initial skin incision of limited extent, the blunt dissector of the invention allows to easily penetrate tissues, dissect tissue plans and create cavities in the body.

The surgical instrument according to the invention comprises a handle and a head having a non-cutting blunt edge in the lower part. The head has an unbroken upper surface which has the general shape of a pear viewed from above. The unbroken upper surface of the head when viewed from the side has the general shape of a duck's bill, the proximal portion being more upwardly prominent than the distal portion, both portions being in smooth continuity. The lower surface of the head is flat. The non-cutting blunt edge is formed and clearly marked at the limit between the unbroken upper surface and the flat lower surface of the head, this non-cutting blunt edge facilitating the non traumatic penetration of the tissues and/or dissection of the tissue plans. This non-cutting blunt edge extends around the distal portion of the duck's bill shape and around substantially the entire periphery of the pear-shaped head. It is not necessary for the non-cutting blunt edge to extend fully to the location of a shaft that can be inserted between the handle and head.

The resulting surgical instrument facilitates dissection and the creation of cavities within the human or animal body, creates less trauma and bleeding in comparison with surgical instruments or techniques of the prior art, increases the accuracy of the procedure and allows to perform it more quickly. As a result, surgical procedures performed with the surgical instrument according to the invention have better patient tolerance under local anesthesia, easier recovery and produce less marked scars.

The surgical instrument according to the invention may be in stainless steel, any other metal or any other material suitable for surgical instruments.

The objects, advantages and other particular features of the present invention will become more apparent upon reading of the following non-restrictive description of preferred embodiments thereof, which are given by way of example only and with reference to the accompanying drawings.

### BRIEF DESCRIPTION OF THE DRAWINGS

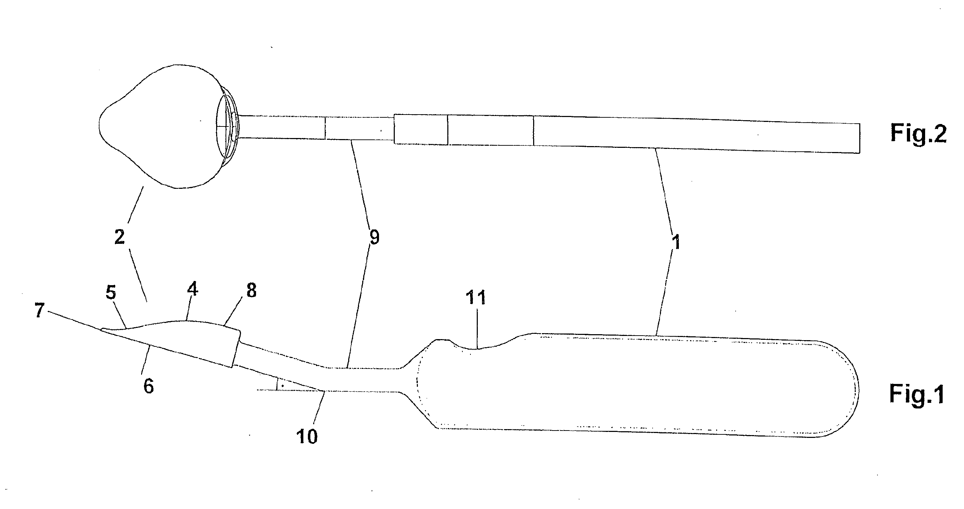

- Fig. 1 is a lateral perspective view of the surgical instrument according to the present invention.
- Fig. 2 is a perspective view from above of the surgical instrument according to the present invention.

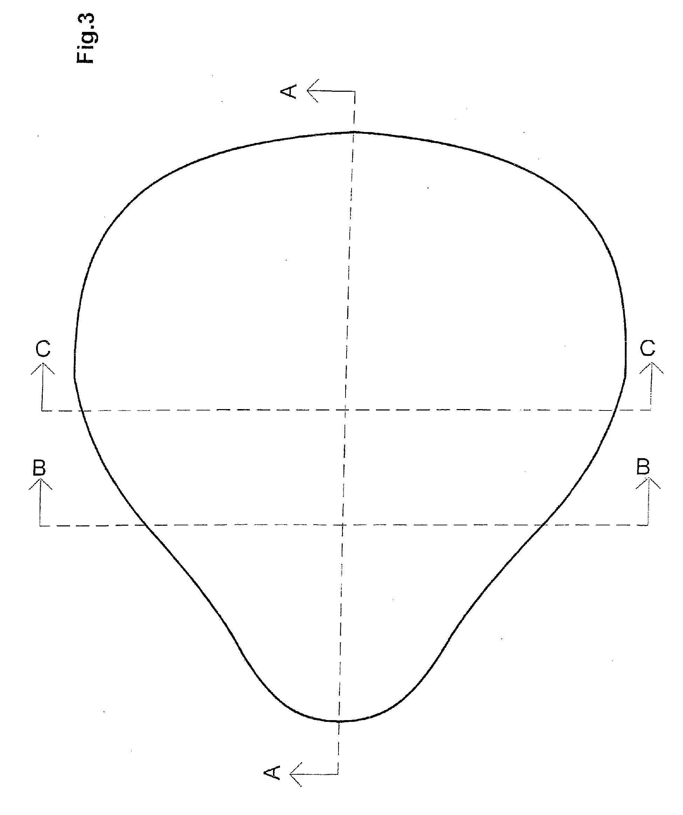

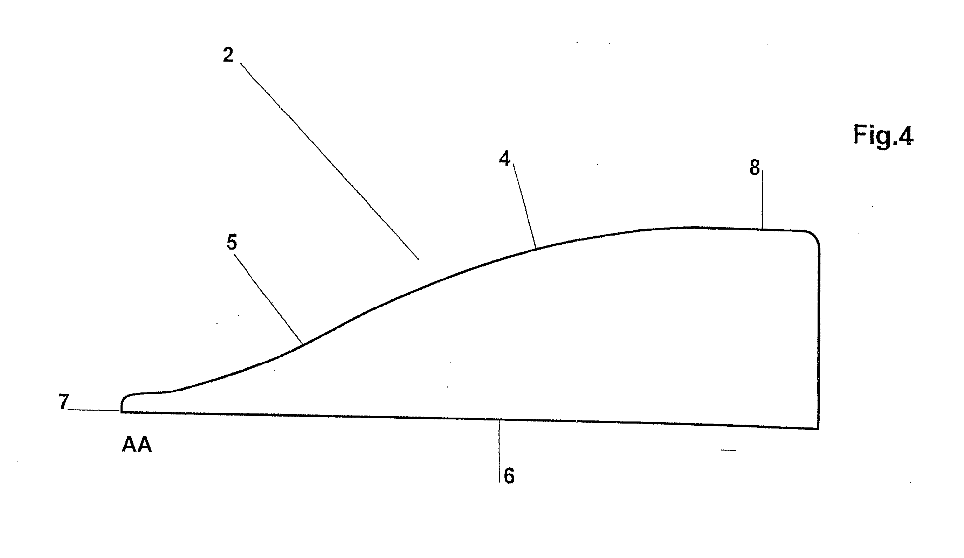

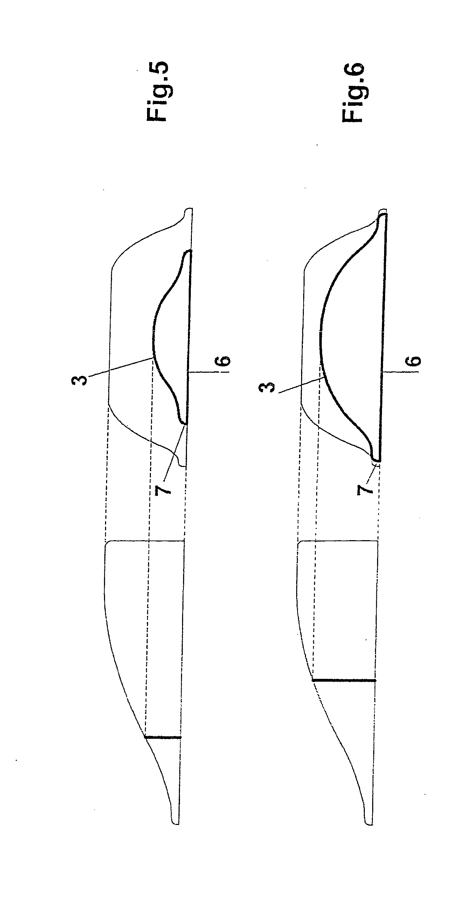

- Fig. 3 is a view from above of the head of the surgical instrument according to the present invention, showing axis A, B, and C used respectively in Fig. 4, 5 and 6.
- Fig. 4 is a longitudinal section through axis A of the head of the surgical instrument according to the present invention.
- Fig. 5 is a transversal section through axis B of the head of the surgical instrument according to the present invention.
- Fig. 6 is a transversal section through axis C of the head of the surgical instrument according to the present invention.

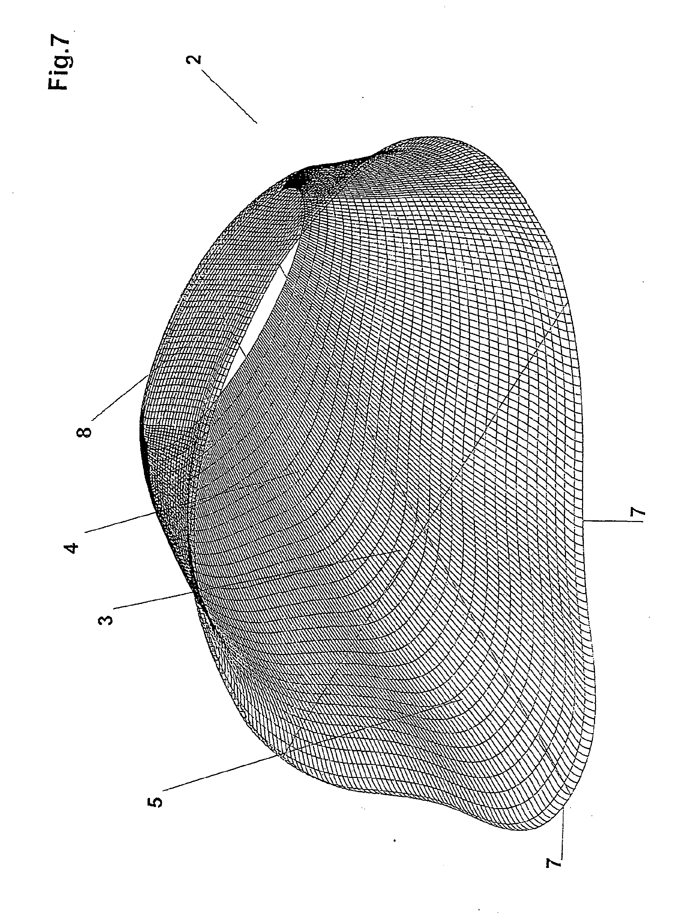

- Fig. 7 is a three-dimensional view of the head of the surgical instrument according to the present invention.

### DESCRIPTION OF THE INVENTION

The present invention provides a blunt dissector for the penetration of tissues, the dissection of tissue plans and the creation of cavities within the human or animal body accurately, in an expedited way and with minimum trauma and risk.

The surgical instrument according to the invention comprises a handle (1) and a head (2), characterized in that:
- Viewed from above, the head (2) has the general shape of a pear;
- The upper surface (3) of the head has the general shape of a duck's bill, the proximal portion (4) being more upwardly prominent than the distal portion (5), both portions being in smooth continuity;
- The lower surface (6) of the head is flat;
- The limit between the upper and the lower surfaces of the head forms a clearly marked, non-cutting blunt edge (7).

In a preferred embodiment of the invention, the proximal portion of the head (4) has a substantially flat area (8) on the top.

In preferred embodiments, the upper surface of the head curves down from the prominent proximal portion (3) to the periphery of the head (2) by a convex curvature followed by a concave curvature.

Also in preferred embodiments, the non-cutting blunt edge (7) is formed by an edge that joins the periphery of the flat lower surface (6) of the head at a right angle or an acute angle approaching a right angle, and that joins the periphery of the upper surface with a more rounded convex curvature.

The thickness of the non-cutting blunt edge (7) depends on the size of the instrument but is generally in the range 0.5mm to 2mm.

In order to put some distance between the handle (1) and the head (2), a shaft (9) may be mounted between said handle and head, the proximal end of the shaft being mounted to the distal end of the handle and the distal end of the shaft being mounted to the proximal portion of the head. In a preferred sub-embodiment, the longitudinal axis of the shaft (9) deviates from being straight at an angle (10) of between 10 and 20 degrees in the vertical plane, so that the distal end of the shaft (9) and the head (2) are pointing up when the handle (1) and the proximal end of the shaft (9) are held horizontally.

In still another embodiment, the surgical instrument according to the invention is further characterized in that the weight of the handle (1) taken individually is at least 400% of the weight of the head (2) taken individually. This weight distribution allows for steady operation by the surgeon.

In order to improve handling, the surgical instrument according to the invention may be further characterized in that the handle (1) has a rounded cut-out on the upper side for improving the grip of the operator's thumb.

The surgical instrument according to the invention may be used for trocar access in laparoscopy, net placement in abdominal wall and hernia repairs, ribs exposure during thoracic surgery, field access in hip prosthesis surgery and other orthopedic procedures. More generally, the surgical instrument according to the invention may be used for any surgical procedure requiring the penetration of tissues, the dissection of tissue plans and/or the creation of cavities in the human or animal body. In particular, the surgical instrument according to the invention may be used for creating cavities in the body in view of inserting foreign bodies. Said foreign bodies may be medical devices, such as cardiac pacemakers, portable catheters, chemotherapy reservoirs, mammary implants, or any other foreign bodies including radio frequency identification tags (RFID tags).

## Claims

1. A surgical instrument for creating a cavity in the human or animal body, comprising a handle (1) and a head (2), having a non-cutting blunt edge (7) in its lower part; **characterized in that**:
- The head (2) has an unbroken upper surface (3) which viewed from above has the general shape of a pear;
- The unbroken upper surface (3) of the head (2) when viewed from the side has the general shape of a duck's bill, the proximal portion (4) being more upwardly prominent than the distal portion (5), both portions being in smooth continuity;
- The lower surface (6) of the head (2) is flat;
and
- The non-cutting blunt edge (7) is formed and clearly marked at the limit between the unbroken upper surface (3) and the flat lower surface (6) of the head (2).

2. The surgical instrument of claim 1, **characterized in that** the proximal portion of the head (2) has a substantially flat area on the top.

3. The surgical instrument of anyone of claims 1 or 2, **characterized in that** a shaft (9) is mounted between the handle (1) and the head (2), the proximal end of the shaft (9) being mounted to the distal end of the handle (1) and the distal end of the shaft (9) being mounted to the proximal portion (4) of the head (2).

4. The surgical instrument of claim 3, **characterized in that** the longitudinal axis of the shaft (9) deviates from being straight to an angle of between 10 and 20 degrees in the vertical plane, so that the distal end of the shaft (9) and the head (2) are pointing up when the handle (1) and the proximal end of the shaft (9) are held horizontally.

5. The surgical instrument of anyone of claims 1 to 4, **characterized in that** the weight of the handle (1) taken individually is at least 400% of the weight of the head (2) taken individually.

6. The surgical instrument of anyone of claims 1 to 5, **characterized in that** the handle (1) has a cut-out on the upper side for improving the grip of the operator's thumb.

7. The surgical instrument of anyone of claims 1 to 6, for creating in the human or animal body a cavity of shape and dimensions corresponding approximately to the shape and dimensions of the instrument's head (2), **characterized in that** the shape and dimensions of the instrument's head (2) correspond to those of a foreign body for insertion in a cavity formed by the instrument.

8. The surgical instrument of claim 7, **characterized in that** the foreign body is a medical device.

9. The surgical instrument of claim 8, **characterized in that** said medical device is a portable catheter.

10. The surgical instrument of claim 8, **characterized in that** said medical device is a chemotherapy reservoir.

11. The surgical instrument of claim 8, **characterized in that** said medical device is a cardiac pacemaker.

12. The surgical instrument of claim 8, **characterized in that** said medical device is a mammary implant.

13. The surgical instrument of claim 7, **characterized in that** said foreign body is a radio frequency identification tag (RFID tag).

## Patentansprüche

1. Chirurgisches Instrument zum Erzeugen einer Kavität in dem menschlichen oder tierischen Körper, umfassend ein Griffstück (1) und einen Kopf (2), der in seinem unteren Teil eine nicht-schneidende, stumpfe Schneide (7) aufweist, **dadurch gekennzeichnet, dass**:
- Der Kopf (2) eine ununterbrochene Oberseite (3) aufweist, die, von oben betrachtet, die allgemeine Form einer Birne aufweist;
- Die ununterbrochene Oberseite (3) des Kopfes (2), von der Seite betrachtet, die allgemeine Form eines Entenschnabels aufweist, wobei der proximale Teil (4) weiter oben hervorstehend ist als der distale Teil(5), wobei beide Teile gleichmäßigen übergehen;
- Die Unterseite (6) des Kopfes (2) flach ist;
und
- Die nicht-schneidende, stumpfe Schneide (7) an der Grenze zwischen der ununterbrochene Oberseite (3) und der flachen Unterseite (6) des Kopfes (2) ausgebildet und klar gekennzeichnet ist.

2. Chirurgisches Instrument nach Anspruch 1, **dadurch gekennzeichnet, dass** der proximale Teil des Kopfes (2) oberseitig einen im Wesentlichen flachen Bereich aufweist.

3. Chirurgisches Instrument nach einem der Ansprüche 1 oder 2, **dadurch gekennzeichnet, dass** ein Schaft (9) zwischen dem Griffstück (1) und dem Kopf (2) angebracht ist, wobei das proximale Ende des Schafts (9) an das distale Ende des Griffstücks (1) angebracht ist und das distale Ende des Schafts (9) an den proximalen Teil (4) des Kopfes (2) angebracht ist.

4. Chirurgisches Instrument nach Anspruch 3, **dadurch gekennzeichnet, dass** die Längsachse des Schafts (9) von einem geradlinigen Verlauf um einem Winkel zwischen 10 und 20 Grad in der vertikalen Ebene abweicht, so dass das distale Ende des Schafts (9) und des Kopfes (2) nach oben zeigen, wenn das Griffstück (1) und das proximale Ende des Schafts (9) horizontal gehalten werden.

5. Chirurgisches Instrument nach einem der Ansprüche 1 bis 4, **dadurch gekennzeichnet, dass** das Gewicht des Griffstücks (1), einzeln betrachtet, mindestens 400% des Gewichts des Kopfes (2), einzeln betrachtet, aufweist.

6. Chirurgisches Instrument nach einem der Ansprüche 1 bis 5, **dadurch gekennzeichnet, dass** das Griffstück (1) an der Oberseite eine Aussparung aufweist, um den Halt des Daumens des Operateurs zu verbessern.

7. Chirurgisches Instrument nach einem der Ansprüche 1 bis 6 zum Erzeugen einer Kavität in dem menschlichen oder tierischen Körper, deren Form und Ausmaße ungefähr der Form und den Ausmaßen des Instrumentenkopfes (2) entsprechen, **dadurch gekennzeichnet, dass** die Form und Ausmaße des Instrumentenkopfes (2) denen eines Fremdkörpers entsprechen, der in eine durch das Instrument gebildete Kavität eingeführt wird.

8. Chirurgisches Instrument nach Anspruch 7, **dadurch gekennzeichnet, dass** der Fremdkörper ein medizinisches Gerät ist.

9. Chirurgisches Instrument nach Anspruch 8, **dadurch gekennzeichnet, dass** das medizinische Gerät ein tragbarer Katheter ist.

10. Chirurgisches Instrument nach Anspruch 7, **dadurch gekennzeichnet, dass** das medizinische Gerät ein chemotherapeutisches Reservoir ist.

11. Chirurgisches Instrument nach Anspruch 7, **dadurch gekennzeichnet, dass** das medizinische Gerät ein Herzschrittmacher ist.

12. Chirurgisches Instrument nach Anspruch 7, **dadurch gekennzeichnet, dass** das medizinische Gerät ein Brustimplantat ist.

13. Chirurgisches Instrument nach Anspruch 7, **dadurch gekennzeichnet, dass** der Fremdkörper ein Radiofrequenz-Identifikations-Tag (RFID-Tag) ist.

## Revendications

1. Instrument chirurgical permettant de créer une cavité dans le corps humain ou animal, comprenant un manche (1) et une tête (2), dotée d'un fil émoussé non coupant (7) dans sa partie inférieure ; **caractérisé en ce que** :
- la tête (2) a une surface supérieure continue (3) qui, vue de dessus, a la forme générale d'une poire ;
- la surface supérieure continue (3) de la tête (2), vue de côté, a la forme générale d'un bec de canard, la partie proximale (4) étant plus proéminente vers le haut que la partie distale (5), les deux parties étant en continuité régulière ;
- la surface inférieure (6) de la tête (2) est plate ; et
- le fil émoussé non coupant (7) est formé et clairement marqué à la limite entre la surface supérieure continue (3) et la surface inférieure plate (6) de la tête (2).

2. Instrument chirurgical selon la revendication 1, **caractérisé en ce que** la partie proximale de la tête (2) a une zone sensiblement plate sur le dessus.

3. Instrument chirurgical selon l'une quelconque des revendications 1 ou 2, **caractérisé en ce qu'**une tige (9) est montée entre le manche (1) et la tête (2), l'extrémité proximale de la tige (9) étant montée sur l'extrémité distale du manche (1) et l'extrémité distale de la tige (9) étant montée sur la partie proximale (4) de la tête (2).

4. Instrument chirurgical selon la revendication 3, **caractérisé en ce que** l'axe longitudinal de la tige (9) dévie d'une droite selon un angle compris entre 10 et 20 degrés dans le plan vertical, de sorte que l'extrémité distale de la tige (9) et la tête (2) pointent vers le haut lorsque le manche (1) et l'extrémité proximale de la tige (9) sont tenus horizontalement.

5. Instrument chirurgical selon l'une quelconque des revendications 1 à 4, **caractérisé en ce que** le poids du manche (1) pris individuellement est d'au moins 400 % du poids de la tête (2) prise individuellement.

6. Instrument chirurgical selon l'une quelconque des revendications 1 à 5, **caractérisé en ce que** le manche (1) a une découpe sur le côté supérieur permettant d'améliorer la préhension du pouce de l'opérateur.

7. Instrument chirurgical selon l'une quelconque des revendications 1 à 6, permettant de créer dans le corps humain ou animal une cavité de forme et de dimensions correspondant approximativement à la forme et aux dimensions de la tête de l'instrument (2), **caractérisé en ce que** la forme et les dimensions de la tête de l'instrument (2) correspondent à celles d'un corps étranger pour une insertion dans une cavité formée par l'instrument.

8. Instrument chirurgical selon la revendication 7, **caractérisé en ce que** le corps étranger est un dispositif médical.

9. Instrument chirurgical selon la revendication 8, **caractérisé en ce que** ledit dispositif médical est un cathéter portable.

10. Instrument chirurgical selon la revendication 8, **caractérisé en ce que** ledit dispositif médical est un réservoir de chimiothérapie.

11. Instrument chirurgical selon la revendication 8, **caractérisé en ce que** ledit dispositif médical est un stimulateur cardiaque.

12. Instrument chirurgical selon la revendication 8, **caractérisé en ce que** ledit dispositif médical est un implant mammaire.

13. Instrument chirurgical selon la revendication 7, **caractérisé en ce que** ledit corps étranger est une étiquette d'identification par radiofréquence (étiquette RFID).
